Europäisches Patentamt

European Patent Office

Office européen des brevets

(11) Veröffentlichungsnummer: **0 116 339**
A1

(12) # EUROPÄISCHE PATENTANMELDUNG

(21) Anmeldenummer: 84101038.2

(22) Anmeldetag: 02.02.84

(51) Int. Cl.³: **C 07 C 11/02**
C 07 C 1/20, B 01 J 21/12

(30) Priorität: 10.02.83 DE 3304478

(43) Veröffentlichungstag der Anmeldung:
22.08.84 Patentblatt 84/34

(84) Benannte Vertragsstaaten:
BE DE FR GB NL

(71) Anmelder: BASF Aktiengesellschaft
Carl-Bosch-Strasse 38
D-6700 Ludwigshafen(DE)

(72) Erfinder: Hoelderich, Wolfgang, Dr.
Mannheimer Strasse 18 c
D-6710 Frankenthal(DE)

(72) Erfinder: Mross, Wolf Dieter, Dr.
Anselm-Feuerbach-Strasse 21
D-6710 Frankenthal(DE)

(72) Erfinder: Schwarzmann, Matthias, Dr.
Carl-Bosch-Strasse 54
D-6703 Limburgerhof(DE)

(54) Verfahren zur Herstellung von C2- bis C4-Olefinen aus Methanol/Dimethylether.

(57) Die Anmeldung betrifft ein Verfahren zur Herstellung von $C_2$- bis $C_4$-Olefinen durch katalytische Umsetzung von Methanol und/oder Dimethylether bei erhöhter Temperatur, wobei als Katalysator ein nicht zeolithisches Aluminosilikat mit Adsorptionseigenschaften, das mit Borsäure getränkt worden ist, eingesetzt wird. Zweckmäßigerweise werden Katalysatoren, die aus 5 bis 70 Gew.-% $B_2O_3$ bestehen, verwendet. Als Ausgangsstoff kann Rohmethanol eingesetzt werden.

EP 0 116 339 A1

Verfahren zur Herstellung von $C_2$- bis $C_4$-Olefinen aus Methanol/Dimethylether

In neuerer Zeit gewinnen Bemühungen, Methanol zur Herstellung von Olefinen zu verwenden, zunehmendes Interesse. Methanol läßt sich aus Kohle, über Kohlevergasung und Herstellung von Synthesegas, mit Hilfe bewährter Technologien leicht herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff beibehalten werden. In den vergangenen Jahren sind daher Verfahren entwickelt worden, die die Herstellung von Olefinen aus Methanol und/oder Dimethylether zum Gegenstand haben.

Ein solches Verfahren ist beispielsweise in der DE-OS 26 15 150 beschrieben. Als Katalysator wird ein Aluminosilikatzeolith ZSM-5 verwendet, der eigentlich ein Aromatisierungskatalysator ist. Die Umsetzung kann aber durch verschiedene Maßnahmen, insbesondere durch die Verkürzung der Verweilzeit, in Richtung Olefinbildung gelenkt werden. Weitere die Olefinbildung begünstigende Parameter sind insbesondere die Verdünnung von Methanol bzw. Dimethylether mit Inertgas bzw. Wasserdampf oder die Verdünnung des Katalysators mit Bindemitteln. Die Erfahrung zeigt, daß hohe Olefinausbeuten nur durch eine sehr starke Verdünnung von Methanol und/oder Dimethylether mit Inertgas oder Wasserdampf zu erzielen sind. In jünster Zeit werden für die Methanolumwandlung in Kohlenwasserstoffe auch acide Heteropolysäuren als Katalysatoren beschrieben. Ein derartiger Katalysator ist beispielsweise Pyrazin-Wolframphosphat (Chem. Lett. 4, 449 f (1981)). Die Herstellung dieser Katalysatoren in definierter Zusammensetzung und in hoher Reinheit ist schwierig. Auch sind die hierbei erzielten $C_2$- bis $C_4$-Olefinausbeuten unbefriedigend.

Es besteht großes Interesse an einem einfachen Verfahren, das die vollständige Umsetzung von Rohmethanol und/oder Dimethylether an einem leicht herzustellenden Katalysator in überwiegend aus $C_2$- bis $C_4$-Olefinen bestehende Kohlenwasserstoffe ermöglicht.

Es wurde nun gefunden, daß man $C_2$- bis $C_4$-Olefine in hoher Ausbeute aus Methanol und/oder Dimethylether durch katalytische Umsetzung bei erhöhter Temperatur erhält, wenn man die Umsetzung in Gegenwart von nicht zeolithischen Aluminiumsilikaten, die Adsorptionseigenschaften aufweisen, und die mit Borsäure dotiert wurden, ausführt.

Vorteilhaft setzt man Katalysatoren ein, bei denen die nicht zeolithischen Aluminiumsilikate, die auch als Trockenmittel Verwendung finden, mit 5 bis 70 Gew.-% $B_2O_3$, bevorzugt 15 bis 50 Gew.-% - bezogen auf das Gesamtgewicht des Katalysators - getränkt werden.

Bei der Durchführung des Verfahrens wird Methanol und/oder Dimethylether bei einem Druck zwischen Normaldruck und etwa 30 bar, vorzugsweise bei 0 bis 1 bar und bei Temperaturen zwischen 300° und 650°C, vorzugsweise bei 400° und 550°C an den oben beschriebenen Katalysatoren umgesetzt. Das Methanol kann einen Wassergehalt bis zu 90 Gew.-% haben, zweckmäßig verwendet man als Ausgangsstoff Rohmethanol, das etwa 20 % Wasser enthält.

Dem Methanol können auch noch andere niedere Alkohole beigemischt sein. Die Belastung des Katalysators, ausgedrückt in WHSV = $h^{-1}$ - g Methanol und/oder Dimethylether pro g Katalysator und Stunde -, wird zweckmäßig so gewählt, daß die Ausgangsstoffe möglichst quantitativ umgesetzt werden, so daß keine Abtrenn- und Rückführprobleme für nicht umgesetzten Dimethylether entstehen. Im allgemeinen soll daher die WHSV im Bereich von 0,5 bis 50 $h^{-1}$, vorzugsweise im Bereich von 2 bis 15 $h^{-1}$ liegen.

Der Vorteil des erfindungsgemäßen Verfahrens liegt in der Anwendung eines Katalysators, der durch eine einfache und wirtschaftliche Herstellungsweise erhalten wird.

Es ist ein weiterer Vorteil der Erfindung, daß die Umsetzung zu $C_2$- bis $C_4$-Olefinen mit Rohmethanol ohne weiteren Zusatz von inertem Verdünnungsmittel wie $N_2$, $H_2$ oder $H_2O$ ausgeführt werden kann und das Methanol vollständig umgesetzt wird.

Die Durchführung des erfindungsgemäßen Verfahrens wird anhand des nachstehenden Beispiels näher erläutert.

Beispiel

21 g Aluminosilikatperlen mit 97 % $SiO_2$- und 3 % $Al_2O_3$-Gehalt und 0,5 bis 1,5 mm Durchmesser (z.B. KC-Trockenperlen WS) werden mit 50 ml Methanol, in dem 14 g $B_2O_3$ gelöst ist, getränkt, danach bei $110^{\circ}C/16$ h getrocknet und anschließend wahlweise bei $500^{\circ}C/5$ h calciniert.

An diesem Katalysator wird unter isothermen Bedingungen in einem Rohrreaktor Rohmethanol mit 20 Gew.-% Wasser bei $550^{\circ}C$ und WHSV = 7,8 $h^{-1}$ bezogen auf eingesetztes $CH_3OH$ quantitativ umgesetzt. Es werden die nachstehenden Ausbeuten bezogen auf eingesetztes $CH_2$ erhalten.

| | | | |
|---|---|---|---|
| $C_2H_4$ | 10,2 % | $CH_4$ | 12,6 % |
| $C_3H_6$ | 45,1 % | $C_2H_6$ | 0,5 % |
| $C_4H_8$ | 23,5 % | $C_3H_8$ | 2,4 % |
| | | $C_4H_{10}$ | 1,4 % |
| | | $C_5^+$-Aliphate | 12,6 % |
| | | $C_6^+$-Aromaten | 1,8 % |

## Patentansprüche

1. Verfahren zur Herstellung von $C_2$- bis $C_4$-Olefinen durch katalytische Umsetzung von Methanol und/oder Dimethylether bei erhöhter Temperatur, dadurch gekennzeichnet, daß man als Katalysator ein nicht zeolithisches Aluminosilikat mit Adsorptionseigenschaften, das mit Borsäure getränkt wird, einsetzt.

2. Verfahren nach Anspruch 1, dadurch gekennzeichnet, daß der Katalysator aus 5 bis 70 Gew.-% $B_2O_3$, bevorzugt 15 bis 50 Gew.-%, besteht.

**0116339**
Nummer der Anmeldung

Europäisches
Patentamt

**EUROPÄISCHER RECHERCHENBERICHT**

EP  84 10 1038

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-4 049 573  (KAEDING) | | C 07 C  11/02<br>C 07 C  1/20<br>B 01 J  21/12 |
| | ----- | | |

RECHERCHIERTE SACHGEBIETE (Int. Cl. ³)

C 07 C  1/00
B 01 J  21/00

Der vorliegende Recherchenbericht wurde für alle Patentansprüche erstellt.

| Recherchenort<br>DEN HAAG | Abschlußdatum der Recherche<br>11-04-1984 | Prüfer<br>VAN GEYT J.J.A. |
|---|---|---|